# EUROPÄISCHE PATENTSCHRIFT

(11) **EP 0 820 974 B1**
(45) Veröffentlichungstag und Bekanntmachung des Hinweises auf die Patenterteilung: **20.12.2000**
(21) Anmeldenummer: 97111021.8
(22) Anmeldetag: 02.07.1997
(51) Int. Cl.: C07C 43/04, C07C 11/02, C07C 41/06, C07C 2/08

(54) **Verfahren zur Herstellung von Alkyltert-butylethern und Di-n-buten aus Feldbutanen**
Process for the preparation of alkyl tert-butyl ethers and di-n-butene from field butanes
Procédé pour la préparation d'éthers alkyls tertio-butyls à partir de butanes naturels

(30) Priorität: 24.07.1996 DE 19629905
(43) Veröffentlichungstag der Anmeldung: 28.01.1998
(73) Patentinhaber: Degussa-Hüls Aktiengesellschaft, 60287 Frankfurt am Main (DE)
(72) Erfinder: Nierlich, Franz, Dr., 45768 Marl (DE); Olbrich, Paul, Dr., 45721 Haltern (DE); Droste, Wilhelm, Dr., 45770 Marl (DE); Müller, Richard, Dr., 45770 Marl (DE); Toetsch, Walter, Dr., 45770 Marl (DE)

(56) Entgegenhaltungen:
- WO-A-90/11268
- FR-A- 2 594 139
- P. R. SARATHY ET AL: "Etherify field butanes. Part 2" HYDROCARBON PROCESSING., Februar 1993, HOUSTON US, Seite 43-46,48,50-51 XP000343972

## Beschreibung

Die Erfindung betrifft ein Verfahren zur Herstellung von Alkyl-tert.butylethern (in der Folge kurz RTBE genannt, wobei R für Alkyl steht) und von Di-n-buten in einer Koppelproduktion aus Feldbutanen, wobei man das iso-Butan in Alkyl-tert.butylether und das n-Butan in Di-n-buten umwandelt und das Mengenverhältnis dieser beiden Produkte regeln kann, indem man das Mengenverhältnis von n-Butan zu iso-Butan durch Isomerisierung entsprechend einstellt.

RTBE werden als Zusatz zu Fahrbenzin zur Erhöhung der Octanzahl verwendet. Man stellt sie durch Anlagerung von Alkanolen an iso-Buten her, die auch als Veretherung bezeichnet wird. Das iso-Buten kann aus vier verschiedenen Quellen stammen: Aus Steamcrackern, Propylenoxid-Anlagen, Erdölraffinerien (d.h. FC-Crackern) und Anlagen zur Dehydrierung von iso-Butan (vgl. R.A.Pogliano et al., Dehydrogenation-Based Ether Production - Adding Value to LPG and Gas Condensate, 1996 Petrochemical Review, DeWitt & Company, Houston Texas). Bei den ersten drei Quellen fällt das iso-Buten als Bestandteil des C₄-Schnitts, also als unmittelbares Nebenprodukt an. Bei der Dehydrierung von iso-Butan ist iso-Buten oft ein mittelbares Nebenprodukt solcher Anlagen, denn der Ausgangsstoff iso-Butan wird ebenfalls als unmittelbares Nebenprodukt in Steamcrackern und Erdölraffinerien oder durch Isomerisierung von n-Butan gewonnen, das seinerseits ein Nebenprodukt in Steamcrackern und Erdölraffinerien ist. Die derzeitige Weltproduktion von RTBE beträgt rund 25 Millionen t/Jahr, mit steigender Tendenz. Der Anfall an Butanen und Butenen als Nebenprodukte in einem bestimmten Cracker oder einer bestimmten Erdölraffinerie ist zu klein, als daß man die "Economies of Scale". die in dem RTBE-Verfahren stecken, voll ausnutzen könnte. Man müßte also iso-Buten und/oder iso-Butan (zum Dehydrieren) aus Crackern und/oder Raffinerien sammeln, um eine RTBE-Anlage mit optimaler Kapazität betreiben zu können. Alternativ könnte man genügend C₄-Schnitt aus solchen Anlagen sammeln und diesen gemeinsam vor Ort auf iso-Buten und iso-Butan aufarbeiten. Beiden Varianten, und insbesondere der zweiten, steht aber entgegen, daß der Transport von Flüssiggasen teuer ist, nicht zuletzt wegen der aufwendigen Sicherheitsmaßnahmen.

Als Dibuten bezeichnet man das Isomerengemisch, das neben höheren Butenoligomeren durch Dimerisierung und/oder Co-Dimerisierung von Butenen, d.h. von n-Buten und/oder iso-Buten, bei der Oligomerisierung von Butenen entsteht. Als Di-n-buten bezeichnet man das Dimerisierungsprodukt von n-Buten, d.h. 1-Buten und/oder 2-Buten. Wesentliche Bestandteile des Di-n-butens sind 3-Methyl-2-hepten, 3,4-Dimethyl-2-hexen und in untergeordnetem Maße n-Octene. Di-iso-buten ist das Isomerengemisch, das durch Dimerisierung von iso-Buten entsteht. Di-iso-buten ist stärker verzeigt als Dibuten und dieses wiederum stärker verzweigt als Di-n-buten.

Dibuten, Di-n-buten und Di-iso-buten sind Ausgangsstoffe für die Herstellung von isomeren Nonanolen durch Hydroformylierung und Hydrierung der so entstehenden C₉-Aldehyde. Ester dieser Nonanole, insbesondere die Phthalsäureester, sind Weichmacher, die in bedeutendem Umfang hergestellt und vor allem für Polyvinylchlorid verwendet werden. Nonanole aus Di-n-buten sind in höherem Maße geradkettig als Nonanole aus Dibuten, die wiederum weniger verzweigt sind als Nonanole aus Di-iso-buten. Ester von Nonanolen aus Di-n-buten haben anwendungstechnische Vorteile gegenüber Estern aus anderen Nonanolen und sind daher besonders gefragt.

Man gewinnt n-Buten für die Dimerisierung, ebenso wie iso-Buten, beispielsweise aus C₄-Schnitten, wie sie in Steamcrackern oder FC-Crackern anfallen. Die C₄-Schnitte werden in der Regel aufgearbeitet, indem man zunächst 1,3-Butadien durch eine selektive Wäsche, z.B. mit N-Methylpyrrolidon, abtrennt. iso-Buten ist ein erwünschter und besonders wertvoller Bestandteil des C₄-Schnitts, weil es sich, allein oder im Gemisch mit anderen C₄-Kohlenwasserstoffen, zu begehrten Produkten chemisch umsetzen läßt, z.B. mit iso-Butan zu hochoctanigem iso-Octan oder mit einem Alkanol zu einem RTBE, insbesondere mit Methanol zu Methyl-tert.butylether (MTBE). Nach der Umsetzung des iso-Butens bleiben die n-Butene sowie n-Butan und iso-Butan zurück. Der Anteil des n-Butens an den Spaltprodukten der Steamcracker bzw. der Erdölraffinerien ist jedoch verhältnismäßig gering. Bei Steamcrackern liegt er in der Größenordnung von knapp 10 Gewichtsprozent, bezogen auf das hauptsächliche Zielprodukt Ethylen. Ein Steamcracker mit der respektablen Kapazität von 600.000 t/Jahr Ethylen liefert also nur rund 60.000 t/Jahr n-Buten. Man könnte zwar dessen Menge (und die der iso-Butene) erhöhen, indem man die rund 15.000 t/Jahr n- und iso-Butan dehydriert, die neben den n-Butenen anfallen. Das empfiehlt sich jedoch nicht, weil Dehydrieranlagen hohe Investitionskosten erfordern und für eine so kleine Kapazität unwirtschaftlich sind.

iso-Buten ist, wie gesagt, ein gefragtes Crackprodukt und steht daher für die Isomerisierung zu n-Buten in der Regel nicht zur Verfügung. Die Menge an n-Butenen, die ein Steamcracker oder eine Erdölraffinerie unmittelbar erzeugt, reicht jedoch nicht aus, um genügend Di-n-buten für eine Nonanol-Anlage zu erzeugen, deren Kapazität so groß ist, daß sie mit den bestehenden großen Anlagen zur Herstellung bedeutender Weichmacheralkohole, wie 2-Ethylhexanol, wirtschaftlich konkurrieren könnte. Propylenoxid-Anlagen sind, wie schon gesagt, noch weniger ergiebig. Man müßte also n-Butene aus verschiedenen Steamcrackern, Raffinerien oder Propylenoxid-Anlagen sammeln (oder C₄-Schnitt aus verschiedenen Quellen auf n-Buten aufarbeiten) und das n-Buten vereint oligomerisieren, um den Bedarf einer hinreichend großen, wirtschaftlichen Nonanol-Anlage an Dibuten zu decken. Der Transport von Flüssiggasen ist jedoch teuer, wie bereits erwähnt.

Es wäre daher erwünscht, wenn man n-Buten und iso-Buten an nur einem Standort ohne Transport über größere Entfernungen in Mengen zur Verfügung stellen könnte, wie sie in einer Koppelproduktion für den Betrieb einer großen, wirtschaftlich vorteilhaften Anlage zur Herstellung von Di-n-buten, beispielsweise mit einer Kapazität von 200.000 bis 800.000 t/Jahr, und einer ebensolchen Anlage zur Herstellung von RTBE, z.B. mit einer Kapazität von 300.000 bis 800.000 t/Jahr, erforderlich sind. Es wäre weiterhin erwünscht, den Verbund dieser Anlagen so zu gestalten, daß man das Mengenverhältnis von n-Buten zu iso-Buten den gewünschten Mengen an Di-n-buten und RTBE entsprechend einstellen kann.

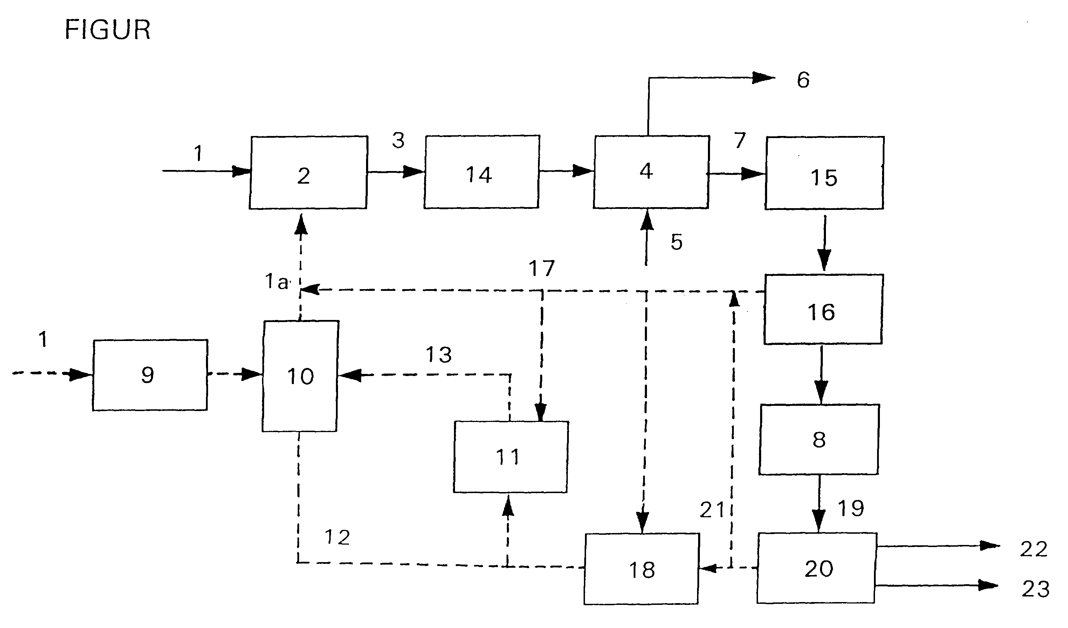

Eine Anlge, die diesen Erfordernissen entspricht, ist mit ihren wesentlichen sowie mit fakultativen Merkmalen in der beigefügten Figur als Bockschema dargestellt.

Die Erfindung ist ein Verfahren zur Herstellung von Alkyl-tert.butylethern und Di-n-buten in einer Koppelproduktion aus Feldbutanen in einem gewünschten Mengenverhältnis, bei dem man
a) die Feldbutane **1** vor dem Eintritt in die Dehydrierungsstufe **2** in der Hydrierstufe **9** Hydrierungsbedingungen unterwirft,
b) die hydrierten Feldbutane in eine Trennstufe **10** führt, der eine Isomerisierungsstufe **11** zugeordnet ist, durch die das n-/iso-Verhältnis nach Maßgabe des gewünschten Mengenverhältnisses von Di-n-buten und Alkyl-tert.butylether eingestellt wird,
c) das so in seinem n-/iso-Verhältnis veränderte Feldbutan **1a** in einer Dehydrierungsstufe **2** zu einem n-Buten und iso-Buten enthaltenden Dehydrierungsgemisch **3** dehydriert,
d) das Dehydrierungsgemisch **3** in die Veretherungsstufe **4** leitet und dort selektiv das iso-Buten mit einem Alkanol **5** zu einem Alkyltert.butylether **6** umsetzt und
e) das restliche Dehydrierungsgemisch **7** in die Oligomerisierungsstufe 8 leitet und dort das n-Buten katalytisch oligomerisiert.

Die Erfindung zeichnet sich durch hohe Flexibilität aus, denn man kann innerhalb der Grenzen, die durch die Kapazitäten der Di-n-buten-Anlage und der RTBE-Anlage gezogen sind, die Mengen an Di-n-buten und an RTBE den Markterfordernissen entsprechend variieren.

Als Feldbutane bezeichnet man die C₄-Fraktion der "feuchten" Anteile des Erdgases sowie der Erdölbegleitgase, die durch Trocknung und Abkühlung auf etwa -30°C in flüssiger Form aus den Gasen abgetrennt werden. Durch Tieftemperaturdestillation gewinnt man daraus die Feldbutane, deren Zusammensetzung je nach Lagerstätte schwankt, die jedoch im allgemeinen etwa 30% iso-Butan und etwa 65% n-Butan enthalten. Weitere Bestandteile sind in der Regel etwa 2 % C_{<4}-Kohlenwasserstoffe und etwa 3% C>4-Kohlenwasserstoffe. Feldbutane können ohne Auftrennung als Einsatzstoffe in Steamcrackern oder als Zusatz zum Fahrbenzin verwendet werden. Sie lassen sich durch fraktionierte Destillation in n-Butan und iso-Butan zerlegen. Iso-Butan wird z.B. in bedeutendem Umfang für die Herstellung von Propylenoxid durch Co-Oxidation von Propylen und iso-Butan sowie als Alkylierungsmittel verwendet, mit dem n-Buten bzw. iso-Buten zu iso-Octan alkyliert wird, das wegen seiner hohen Octanzahl als Zusatz zum Fahrbenzin geschätzt wird. n-Butan hat dagegen weniger bedeutende Verwendungen gefunden. Es dient z.B. als Butangas zu Heizzwecken oder wird in vergleichsweise kleinen Mengen beispielsweise zur Herstellung von Polymeren oder Copolymeren oder von Maleinsäureanhydrid durch Luftoxidation verwendet. Früher wurde n-Butan auch über die Stufe des n-Butens zu 1,3-Butadien dehydriert, doch ist dieses Verfahren inzwischen unwirtschaftlich geworden.

Weil iso-Butan der begehrtere Bestandteil des Feldbutans ist, wird n-Butan in großem Maßstab zu iso-Butan isomerisiert (vgl. z.B. R.A. Pogliano et al., Dehydrogenation-based Ether Production, 1996 Petrochemical Review, DeWitt & Company, Houston, Texas, Butamer^{(R)}-Verfahren, Seite 6; sowie S.T.Bakas, F.Nierlich et al., Production of Ethers from Field Butanes and Refinery Streams, AIChE Summer Meeting. 1990, San Diego, Califörnia, Seite 11). Es lag daher nicht im Trend der Technik, ein Verfahren zu entwickeln, das n-Butan als solches nutzt oder gar iso-Butan in n-Butan umwandelt, um daraus mehr Di-n-buten herzustellen.

Das Verfahren nach der Erfindung wird in zwei aufeinanderfolgenden Teilschritten - (A) Herstellung von RTBE und (B) Herstellung von Di-n-buten - durchgeführt. Im Prinzip ist die Reihenfolge dieser Teilschritte beliebig, doch ist es vorteilhaft, zunächst RTBE und dann Di-n-buten herzustellen, weil iso-Buten ebenfalls oligomerisierungsaktiv ist. Das so entstehende Di-iso-buten ist, wie schon erwähnt, stärker verzweigt und führt deshalb zu Isononanolen mit schlechteren anwendungstechnischen Eigenschaften.

### (A) Herstellung von RTBE

Die Feldbutane 1 bzw. die durch Isomerisierung in ihrer Zusammensetzung veränderten Feldbutane **1a** (siehe Abschnitt (C)) werden in die Dehydrierungsstufe 2 geleitet, die ein wesentliches Merkmal des Verfahrens nach der Erfindung ist. Dort werden die Feldbutane zu einem n-Buten und iso-Buten enthaltenden Dehydrierungsgemisch 3 dehydriert. Die Dehydrierung ist eine Co-Dehydrierung von n-Butan und iso-Butan. Es ist bemerkenswert, daß die Dehydrierung der Feldbutane, die ein Gemisch aus Bestandteilen mit unterschiedlichem Dehydrierverhalten sind, so glatt gelingt. Die Verfahrensbedingungen entsprechen weitgehend denjenigen, wie sie für n-Butan und für iso-Butan bekannt sind. So beschreiben S.T.Bakas, F.Nierlich et al., loc. cit., Seite 12 ff., das Oleflex^{(R)}-Verfahren, das allgemein für die Herstellung von leichten Olefinen geeignet ist und mit dem z.B. iso-Butan mit einer Selektivität von 91 bis 93 % zu iso-Buten dehydriert werden kann. Weitere einschlägige Veröffentlichungen sind die von G.C.Sturtevant et al., Oleflex - Selective Production of Light Olefins, 1988 UOP Technology Conference, sowie EP 0 149 698. Man führt die Dehydrierung zweckmäßig in der Gasphase an fest angeordneten oder fluidisierten Katalysatoren durch, z.B. an Chrom(III)-oxid oder vorteilhaft an Platinkatalysatoren mit Aluminiumoxid oder Zeolithen als Träger. Die Dehydrierung findet im allgemeinen bei Temperaturen von 400 bis 800°C. vorteilhaft von 550 bis 650°C statt. Man arbeitet in der Regel bei Atmosphärendruck oder leicht erhöhtem Druck bis zu 3 bar. Die Verweilzeit in der Katalysatorschicht beträgt, je nach Katalysator, Temperatur und angestrebtem Umsatzgrad, im allgemeinen zwischen 1 und 60 Minuten. Der Durchsatz liegt dementsprechend in der Regel zwischen 0,6 und 36 kg n-Butan und iso-Butan (als Gemisch) je m³ Katalysator und Stunde.

Es ist zweckmäßig, die Dehydrierung nur soweit zu treiben, daß im Dehydrierungsgemisch 3 etwa 50% des n-Butans und des iso-Butans unverändert bleiben. Zwar kann man bei höherer Temperatur höhere Umsatzgrade erreichen. Dann laufen aber in steigendem Maße Crackreaktionen ab, die die Ausbeute vermindern und, infolge von Koksablagerungen. die Lebensdauer des Dehydrierungskatalysators herabsetzen. Die optimalen Kombinationen der Reaktionsbedingungen, die zu den gewünschten Umsatzgraden führen, wie Art des Katalysators, Temperatur und Verweilzeit, lassen sich unschwer durch orientierende Versuche ermitteln.

Das Dehydrierungsgemisch **3** enthält in der Regel 90 bis 95 Gew.-% C4-Kohlenwasserstoffe und daneben Wasserstoff sowie niedriger- und höhersiedende Anteile. Es wird vor der Oligomerisierung zweckmäßig vorgereinigt, und zwar in einer ersten Reinigungsstufe (in der Figur nicht dargestellt) und in einer Selektivhydrierungsstufe **14**. In der ersten Reinigungsstufe werden die C₄-Fraktion und die höhersiedenden Anteile aus der Gasphase herauskondensiert. Das Kondensat wird unter Druck destilliert, wobei mitkondensierte, gelöste C_{<4}-Kohlenwasserstoffe über Kopf gehen. Aus den höhersiedenden Anteilen gewinnt man in einer weiteren Destillation als Hauptprodukt die gesättigten und ungesättigten C₄-Kohlenwasserstoffe, die in das weitere Verfahren gehen, und als Rückstand die vergleichsweise kleine Menge an C_{>4} -Kohlenwasserstoffen.

Die C₄-Kohlenwasserstoffe enthalten im allgemeinen geringe Mengen, z.B. 0,01 bis 5 Volumenprozent, Diene, wie Propadien und insbesondere 1,3-Butadien. Es ist empfehlenswert, diese Diene zu entfernen, da sie selbst in deutlich geringeren Mengen später den Katalysator in der Oligomerisierungsstufe **8** schädigen können. Ein geeignetes Verfahren ist die Selektivhydrierung **14**, die zudem den Anteil des erwünschten n-Butens erhöht. Die Selektivhydrierung wurde z.B. von F.Nierlich et al. in Erdöl & Kohle, Erdgas, Petrochemie. **1986**,, Seite 73 ff. beschrieben. Sie wird in flüssiger Phase mit vollständig gelöstem Wasserstoff in stöchiometrischen Mengen durchgeführt. Als selektive Hydrierkatalysatoren eignen sich z.B. Nickel und insbesondere Palladium auf einem Träger, z.B. 0,3 Gewichtsprozent Palladium auf Aktivkohle oder vorzugsweise auf Aluminiumoxid. Eine geringe Menge Kohlenmonoxid im ppm-Bereich fördert die Selektivität der Hydrierung des 1,3-Butadiens zum Monoolefin und wirkt der Bildung von Polymeren, dem sogenannten "green oil" entgegen, die den Katalysator inaktivieren. Das Verfahren arbeitet im allgemeinen bei Raumtemperatur oder erhöhter Temperatur bis zu 60°C und unter erhöhten Drücken, die zweckmäßig bis zu 20 bar betragen. Der Gehalt an 1,3-Butadien im Dehydrierungsgemisch wird auf diese Weise auf Werte von <1 ppm gesenkt. Vorteilhaft wird die selektive Hydrierung unter hydroisomerisierenden Bedingungen durchgeführt. Dadurch wird gleichzeitig 1-Buten zu 2-Buten isomerisiert, das im Gegensatz zu 1-Buten in der später zu beschreibenden Trennstufe **16** durch Destillation von n-Butan/iso-Butan getrennt werden kann. Zu Einzelheiten der selektiven Hydrierung unter hydroisomerisierenden Bedingungen siehe z.B. F.Nierlich, Integrated Tert.Butyl Alcohol/Di-n-Butene Production from FCC C₄'s, Erdöl, Erdgas, Kohle 103 (11), Seiten 486 ff., 1989.

Da die Diene zwar die spätere Oligomerisierung, aber weniger die Veretherung stören, kann die Selektivhydrierungsstufe **14** auch nach der Veretherungsstufe **4** im Strom des restlichen Dehydrierungsgemisches **7** angeordnet sein, vor oder vorzugsweise nach der später zu beschreibenden Reinigungsstufe **15.** Diese Anordnung erlaubt es gegebenenfalls, den Reaktor der Selektivhydrierungsstufe **14** kleiner auszulegen, weil das Volumen des restlichen Dehydrierungsgemisches **7** nach der Abtrennung des iso-Butens in der Veretherungsstufe 4 natürlich kleiner ist als das des Dehydrierungsgemisches **3**.

Das Dehydrierungsgemisch **3** wird, gegebenenfalls nach Vorreinigung und Selektivhydrierung, in die Veretherungsstufe **4** geleitet, die ein wesentliches Merkmal des Verfahrens nach der Erfindung ist. Dort wird das darin enthaltene iso-Buten in an sich bekannter Weise mit einem Alkanol 5 umgesetzt (siehe z.B. Methyl-Tert-Butyl Ether, Ullmanns Enzyclopedia of Industrial Chemistry, Band A 16, Seiten 543 ff., VCH Verlagsgesellschaft, Weinheim). Bevorzugte Alkanole sind diejenigen mit 1 bis 6 Kohlenstoffatomen, wie Ethanol, Isopropanol, Isobutanol und insbesondere Methanol. Da n-Buten erheblich weniger reaktiv ist, findet eine selektive Veretherung statt, die praktisch nur iso-Buten verbraucht. Die Reaktion läuft in Flüssigphase oder in Gasflüssigphase ab, im allgemeinen bei einer Temperatur von 50 bis 90°C und unter einem Druck, der sich bei der jeweiligen Temperatur einstellt. Zweckmäßig arbeitet man mit einem geringen stöchiometrischen Überschuß an Methanol, wodurch die Selektivität der Umsetzung des iso-Butens erhöht und dessen Dimerisierung zurückgedrängt wird. Als Katalysator wird z.B. ein saurer Bentonit oder vorteilhaft ein großporiger saurer Ionenaustauscher verwendet.

Aus dem Reaktionsgemisch der Veretherungsstufe **4** trennt man durch Destillation das gasförmige restliche Dehydrierungsgemisch **7** und das überschüssige Alkanol von dem gebildeten RTBE **6** ab. Im Falle von MTBE bilden das restliche Dehydrierungsgemisch **7** und Methanol ein Azeotrop. Das Azeotrop wird mit Wasser gewaschen und in eine wässerige Phase und restliches Dehydrierungsgemisch **7** getrennt. Die wässerige Phase wird auf Methanol, das in die Veretherung zurückgeführt wird, und auf Wasser aufgearbeitet, das wieder zur Wäsche benutzt wird. Das restliche Dehydrierungsgemisch **7** geht in die Herstellung von Di-n-buten.

### (B) Herstellung von Di-n-buten

Ausgangsstoff hierfür ist das im restlichen Dehydrierungsgemisch **7** enthaltene n-Buten. Wenn vor der Veretherungsstufe **4** keine Selektivhydrierung **14** vorgesehen war, so sollte sie jetzt stattfinden, vor oder nach und vorteilhafter nach der Reinigungsstufe **15.** Deren wesentlicher Bestandteil ist ein Molekularsieb, an dem andere für den Oligomerisierungskatalysator schädliche Stoffe entfernt werden, wodurch dessen Lebensdauer weiter erhöht wird. Zu diesen schädlichen Stoffen zählen Sauerstoff- und Schwefelverbindungen. Die Reinigung mittels Molekularsieben ist z.B. von F.Nierlich et al. in EP-B1 0 395 857 beschrieben worden. Man verwendet zweckmäßig ein Molekularsieb mit einem Porendurchmesser von 4 bis 15 Angström, vorteilhaft von 7 bis 13 Angström. In manchen Fällen ist es aus wirtschaftlichen Gründen zweckmäßig, das restliche Dehydrierungsgemisch 7 nacheinander über Molekularsiebe mit unterschiedlichen Porengrößen zu leiten. Das Verfahren kann in der Gasphase, in Flüssigphase oder in Gasflüssigphase durchgeführt werden. Der Druck beträgt dementsprechend im allgemeinen 1 bis 200 bar. Man arbeitet zweckmäßig bei Raumtemperatur oder erhöhten Temperaturen bis zu 200 °C.

Die chemische Natur der Molekularsiebe ist weniger wichtig als ihre physikalische Beschaffenheit, d.h. insbesondere die Porengröße. Man kann also die verschiedensten Molekularsiebe einsetzen, sowohl kristalline, natürliche Aluminiumsilikate, z.B. Schichtgittersilikate, als auch synthetische Molekularsiebe, z.B. solche mit Zeolithstruktur. Zeolithe vom A-, X- und Y-Typ sind u.a. von Bayer AG. Dow Chemical Co., Union Carbide Corporation, Laporte Industries Ltd. und Mobil Oil Co. erhältlich. Für das Verfahren eignen sich auch solche synthetische Molekularsiebe, die neben Aluminium und Silicium noch andere, durch Kationenaustausch eingeführte Atome enthalten, wie Gallium, Indium oder Lanthan sowie Nickel, Kobalt, Kupfer, Zink oder Silber. Weiterhin sind synthetische Zeolithe geeignet, bei denen neben Aluminium und Silicium noch andere Atome, wie Bor oder Phosphor, durch Mischfällung in das Gitter eingebaut worden sind.

Aus dem gegebenenfalls durch Selektivhydrierung **14** und/oder Behandlung mit einem Molekularsieb **15** gereinigten restlichen Dehydrierungsgemisch **7** wird vorteilhaft in der Trennstufe **16** n-Buten von den anderen gasförmigen Bestandteilen (Restgas I **17**). wie iso-Butan und in der Veretherungsstufe **4** nicht umgesetztem iso-Buten, abgetrennt und in die Oligomerisierungsstufe **8** geführt, die ein wesentlicher Teil des Verfahrens nach der Erfindung ist. Diese Auftrennung des restlichen Dehydrierungsgemisches **7** vor der Oligomerisierung ist zweckmäßig, weil sonst die Oligomerisierungsstufe **8** mit unnötig großen Stoffmengen belastet wird und zudem unerwünschte Co-Oligomere aus n-Buten und iso-Buten entstehen.

Die Oligomerisierung wird in an sich bekannter Weise durchgeführt, wie z.B. von F.Nierlich in Oligomerization for Better Gasoline, Hydrocarbon Processing, **1992** (2), Seite 45 ff., oder von F.Nierlich et al. in dem bereits erwähnten EP-B1 0 395 857 beschrieben wurde. Man arbeitet im allgemeinen in flüssiger Phase und wendet als homogenen Katalysator z.B. ein System an, das aus Nickel-(II)-octoat. Ethylaluminiumchlorid und einer freien Fettsäure besteht (DE-PS 28 55 423), oder benutzt vorzugsweise einen der zahlreichen bekannten, fest angeordneten oder im Oligomerisierungsgemisch suspendierten Katalysatoren auf Basis von Nickel und Silicium. Die Katalysatoren enthalten oftmals zusätzlich Aluminium. So beschreibt die DD-PS 160 037 die Herstellung eines Nickel und Aluminium enthaltenden Fällungskatalysators auf Siliciumdioxid als Trägermaterial. Andere brauchbare Katalysatoren erhält man, indem man auf der Oberfläche der Trägermaterialien befindliche positiv geladene Teilchen, wie Protonen oder Natriumionen, gegen Nickelionen austauscht. Dies gelingt bei den verschiedensten Trägermaterialien, wie amorphem Aluminiumsilikat (R.Espinoza et al., Appl.Kat., **31** (1987). Seiten 259-266: kristallinem Aluminiumsilikat (DE-PS 20 29 624); Zeolithen vom ZSM-Typ (NL-PS 8 500 459); einem X-Zeolith (DE-PS 23 47 235); X- und Y-Zeolithen (A.Barth et al., Z.Anorg.Allg.Chem. **521**, (1985) Seiten 207-214); und einem Mordenit (EP-A 0 233 302).

Die Oligomerisierung erfolgt zweckmäßig, je nach Katalysator, bei 20 bis 200°C und unter Drücken von 1 bis 100 bar. Die Reaktionszeit (oder Kontaktzeit) beträgt im allgemeinen 5 bis 60 Minuten. Die Verfahrensparameter, insbesondere die Art des Katalysators, die Temperatur und die Kontaktzeit, werden so aufeinander abgestimmt, daß der gewünschte Oligomerisierungsgrad, d.h. vorwiegend eine Dimerisierung, erreicht wird. Dazu darf man die Reaktion natürlich nicht auf vollen Umsatz fahren, sondern strebt zweckmäßig Umsätze von 30 bis 70 % pro Durchgang an. Die optimalen Kombinationen der Verfahrensparameter lassen sich durch orientierende Versuche ohne Schwierigkeiten ermitteln.

Aus dem Oligomerisierungsgemisch **19** wird in der Trennstufe 20 durch Destillation das Restgas II **21** abgetrennt. Es kann in die Dehydrierungsstufe **2** zurückgeführt werden oder in die Isomerisierungsstufe **11** geleitet werden, sofern diese vorhanden und in Betrieb ist. Schließlich kann das Restgas II **21** auch in die Hydrierstufe **18** geleitet werden, deren Funktion in der Folge erläutert wird. Die Alternativen für die Behandlung des Restgases II **21** sind in der Figur durch gestrichelte Linien angedeutet. Wenn in der Oligomerisierungsstufe **8** ein Katalysator vom Typ der erwähnten flüssigen Katalysatoren verwendet wurde, sollten die Restgase II **21** zuvor zur Schonung des Dehydrierungskatalysators bzw. des Isomerisierungskatalysators gereinigt werden. Man behandelt zunächst das Oligomerisierungsgemisch **19** mit Wasser, um die Katalysatorbestandteile zu extrahieren. Das abgetrennte Restgas II **21** wird dann mit einem geeigneten Molekularsieb getrocknet, wobei auch andere Nebenbestandteile abgetrennt werden. Danach entfernt man durch Hydrierung, z.B. an Palladiumkatalysatoren, mehrfach ungesättigte Verbindungen, wie Butine, und führt schließlich das so gereinigte Restgas II **21** in die Dehydrierungsstufe **2** oder in die Isomerisierungsstufe **11**. Diese Reinigungsmaßnahmen für das Restgas II **21** erübrigen sich, wenn ein fester Oligomerisierungskatalysator verwendet wird.

Aus der verbleibenden flüssigen Phase des Oligomerisierungsgemisches **19** werden in der Trennstufe **20** weiterhin durch fraktionierte Destillation Di-n-buten **22** sowie trimeres n-Buten **23**, d.h. isomere Dodecene, abgetrennt. Das Hauptprodukt Di-n-buten ist unmittelbar für die Herstellung von Nonanolen geeignet. Die Dodecene **23** sind ein erwünschtes Nebenprodukt. Sie können hydroformyliert, die Hydroformylierungsprodukte hydriert und die so erhaltenen Tridecanole oxethyliert werden, wodurch man wertvolle Waschrohstoffe erhält.

Das in der Trennstufe 16 anfallende Restgas I **17** kann in die Dehydrierungsstufe **2** zurückgeführt werden, sofern die Feldbutane 1 ohne Veränderung des n-/iso-Verhältnisses durch Isomerisierung direkt dehydriert werden. Ist eine Isomerisierungsstufe **11** vorhanden und in Betrieb, so kann das Restgas I **17** direkt oder über die Hydrierstufe **18** in die Isomerisierungsstufe **11** geleitet werden. Die Alternativen für die Behandlung des Restgases I **17** sind in der Figur wiederum durch gestrichelte Linien dargestellt.

### (C) Variation der Di-n-buten- und RTBE-Mengen

Wie zuvor erwähnt, ist es zweckmäßig, dem Verfahren eine Isomerisierungsstufe **11** anzugliedern, weil man dadurch das Verhältnis der Mengen an Di-n-buten und RTBE (Produktverhältnis) variieren kann. Die Variationsmöglichkeiten sind nur durch die Kapazitäten der Di-n-buten- und der RTBE-Anlage begrenzt. Mit Rücksicht auf die Investitionskosten wird man wohl selten beide Anlagen so groß auslegen, daß der gesamte zur Verfügung stehende Feldbutan-Strom in nur einer der Anlagen verarbeitet werden kann, während die andere Anlage stillliegt. Trotzdem bringt die Isomerisierungsstufe **11** die Möglichkeit, innerhalb der gegebenen Grenzen flexibel auf die Anforderungen des Marktes zu reagieren.

Will man das vorgegebene n-/iso-Verhältnis der Feldbutane **1** ändern, so leitet man sie zweckmäßig zunächst in eine Hydrierstufe **9**, sofern sie ungesättigte Verbindungen enthalten. Die ungesättigten Verbindungen werden dort hydriert und können dann nicht mehr den Katalysator der Isomerisierungsstufe **11** schädigen. Die Hydrierung erfolgt in sich bekannter Weise (siehe z.B. K.H.Walter et al. in The Hüls Process for Selective Hydrogenation of Butadiene in Crude C₄'s, Development and Technical Application, DGKM-Tagung, Kassel, November 1993). Man arbeitet also zweckmäßig in flüssiger Phase und, je nach Katalysator, bei Raumtemperatur oder erhöhter Temperatur bis zu 90°C und unter einem Druck von 4 bis 20 bar, wobei der Partialdruck des Wasserstoffs 1 bis 15 bar beträgt. Man verwendet die für die Hydrierung von Olefinen üblichen Katalysatoren, z.B. 0.3 % Palladium auf Aluminiumoxid.

Die hydrierten Feldbutane **1** werden in die Trennstufe **10** geleitet, deren wesentlicher Bestandteil eine wirksame, bei tiefer Temperatur und/oder erhöhtem Druck betriebene Kolonne ist. Wenn mehr Alkyl-tert.butylether hergestellt werden soll, als dem iso-Butan-Anteil des Feldbutans **1** entspricht, wird eine dem gewünschten Produktverhältnis entsprechende Menge n-Butan **12** im Seitenstrom abgezogen (als Sumpfprodukt fallen die C_{>4}-Kohlenwasserstoffe an) und in die Isomerisierungsstufe **11** geführt. Der fakultative Charakter dieser Maßnahme ist in der Figur durch eine gestrichelte Linie angedeutet. In der Isomerisierungsstufe **11** wird n-Butan maximal bis zum Gleichgewicht, das je nach der Temperatur bei 40 bis 55 % n-Butan und 60 bis 45 % iso-Butan liegt, in iso-Butan umgewandelt. Das Isomerisierungsgemisch **13** geht in die Trennstufe **10** zurück. Im Ergebnis wird also der Dehydrierungsstufe **2** ein Feldbutan zugeführt, dessen Anteil an iso-Butan gegenüber dem Feldbutan **1** erhöht ist.

Wenn mehr Di-n-buten hergestellt werden soll, als dem n-Butan-Anteil des Feldbutans **1** entspricht, leitet man zweckmäßig das iso-Butanreiche Restgas I **17** aus der Trennstufe **16** vollständig oder zum Teil. entweder direkt oder über die Hydrierstufe **18** in die Isomerisierungsstufe**11.** In diesem Falle wird das Restgas II **21** direkt in die Dehydrierstufe **2** geführt. Im Ergebnis wird dann der Dehydrierungsstufe **2** ein Feldbutan zugeführt, dessen Anteil an n-Butan gegenüber dem Feldbutan **1** erhöht ist.

Die Isomerisierung von n- und iso-Butan ist eine bekannte Reaktion. Man arbeitet im allgemeinen in der Gasphase bei einer Temperatur von 150 bis 230°C, unter einem Druck von 14 bis 30 bar und mit einem Platin-Katalysator auf Aluminiumoxid als Träger, dessen Selektivität durch Dotierung mit einer Chlorverbindung, wie Tetrachlorkohlenstoff, noch verbessert werden kann. Man setzt vorteilhaft eine kleine Menge Wasserstoff zu, um einer Dehydrierung entgegenzuwirken. Die Selektivität der Isomerisierung ist hoch, Crackung zu kleineren Bruchstücken findet nur in untergeordnetem Maße (ca. 2 %) statt (siehe z.B. H.W.Grote, Oil and Gas Journal, **56** (13). Seite 573 ff. (1958)). Die Ausbeuten an dem gewünschten Isomeren ist dementsprechend hoch.

Das Isomerisierungsgemisch **13** wird in die Trennstufe 10 zurückgeführt. aus der ein Feldbutan **1a** mit gegenüber dem ursprünglichen Feldbutan **1** zweckentsprechend veränderten n-/iso-Verhältnis in die Dehydrierungsstufe **2** gelangt.

## Patentansprüche

1. Verfahren zur Herstellung von Alkyl-tert.butylethern und Di-n-buten in einer Koppelproduktion aus Feldbutanen in einem gewünschten Mengenverhältnis, dadurch gekennzeichnet,
daß man
a) die Feldbutane **1** vor dem Eintritt in die Dehydrierungsstufe **2** in der Hydrierstufe **9** Hydrierungsbedingungen unterwirft,
b) die hydrierten Feldbutane in eine Trennstufe **10** führt, der eine Isomerisierungsstufe **11** zugeordnet ist, durch die das n-/iso-Verhältnis nach Maßgabe des gewünschten Mengenverhältnisses von Di-n-buten und Alkyl-tert.butylether eingestellt wird,
c) das so in seinem n-/iso-Verhältnis veränderte Feldbutan **1a** in einer Dehydrierungsstufe **2** zu einem n-Buten und iso-Buten enthaltenden Dehydrierungsgemisch **3** dehydriert,
d) das Dehydrierungsgemisch **3** in die Veretherungsstufe **4** leitet und dort selektiv das iso-Buten mit einem Alkanol **5** zu einem Alkyl-tert.butylether **6** umsetzt und
e) das restliche Dehydrierungsgemisch **7** in die Oligomerisierungsstufe **8** leitet und dort das n-Buten katalytisch oligomerisiert.

2. Verfahren nach Anspruch 1,
dadurch gekennzeichnet,
daß man zwischen der Dehydrierungsstufe **2** und der Veretherungsstufe **4** oder der Veretherungsstufe **4** und der Oligomerisierungsstufe **8** eine Selektivhydrierungsstufe **14** und/oder eine Reinigungsstufe **15** mit Molekularsieb anordnet.

3. Verfahren nach einem der Ansprüche 1 oder 2,
dadurch gekennzeichnet,
daß man nach der Veretherungsstufe **4** aus dem restlichen Dehydrierungsgemisch **7** in einer Trennstufe **16** n-Buten von dem Restgas I **17** abtrennt und dieses in die Dehydrierungsstufe **2** oder, gegebenenfalls über eine Hydrierstufe **18**, in die Isomerisierungsstufe **11** führt.

4. Verfahren nach einem der Ansprüche 1 bis 3,
dadurch gekennzeichnet,
daß man aus dem Oligomerisierungsgemisch 19 in einer Trennstufe 20 das Restgas II 21 abtrennt und in die Dehydrierungsstufe 2 oder, gegebenenfalls über eine Hydrierstufe 18, in die Isomerisierungsstufe 11 leitet.

5. Verfahren nach einem der Ansprüche 1 bis 4,
dadurch gekennzeichnet,
daß man als Alkanol Ethanol, Isopropanol, Isobutanol oder insbesondere Methanol verwendet.

## Claims

1. A process for preparing alkyl tert-butyl ether and di-n-butene in a desired ratio in a coupled production from field butanes, characterized in that
a) the field butanes 1, prior to entry into the dehydrogenation stage 2, are subjected to hydrogenation conditions in the hydrogenation stage 9,
b) the hydrogenated field butanes are passed into a separation stage 10, with which an isomerization stage 11 is associated by which the n-/iso-ratio is set in accordance with the desired ratio of di-n-butene to alkyl tert-butyl ether,
c) the field butanes la thus altered in their n-/iso-ratio are dehydrogenated in a dehydrogenation stage 2 to give a dehydrogenation mixture 3 containing n-butene and isobutene,
d) the dehydrogenation mixture 3 is passed into the etherification stage 4 and there the isobutene is selectively reacted with an alkanol 5 to give an alkyl tert-butyl ether 6, and
e) the residual dehydrogenation mixture 7 is passed into the oligomerization stage 8 and there the n-butene is catalytically oligomerized.

2. A process according to claim 1, characterized in that a selective hydrogenation stage 14 and/or a purification stage 15 having a molecular sieve is arranged between the dehydrogenation stage 2 and the etherification stage 4 or between the etherification stage 4 and the oligomerization stage 8.

3. A process according to either of claims 1 and 2, characterized in that n-butene from the residual dehydrogenation mixture 7 is separated off from the residual gas I 17 downstream of the etherification stage 4 in a separation stage 16 and this residual gas is conducted into the dehydrogenation stage 2 or, if desired via a hydrogenation stage 18, into the isomerization stage 11.

4. A process according to any one of claims 1 to 3, characterized in that the residual gas II 21 from the oligomerization mixture 19 is separated off in a separation stage 20 and passed into the dehydrogenation stage 2 or, if desired via a hydrogenation stage 18, into the isomerization stage 11.

5. A process according to any one of claims 1 to 4, characterized in that ethanol, isopropanol, isobutanol or, in particular, methanol is used as alkanol.

## Revendications

1. Procédé de fabrication de terbutyléthers d'alkyle et de di-n-butène dans une production jumelée à partir de butanes de champ, dans un rapport quantitatif désiré,
caractérisé en ce que
a) ces butanes de champ (1) avant l'entrée à l'étape de déshydrogénation (2), sont soumis à l'étape d'hydrogénation (9) aux conditions d'hydrogénation,
b) on mène les butanes de champ hydrogénés à une étape de séparation (10) qui est coordonnée à une étape d'isomérisation (11), grâce à laquelle le rapport n/iso est réglé d'après la mesure du rapport désiré quantitatif de di-n-butène et de terbutyléther d'alkyle,
c) on déshydrogène le butane de champ (la) modifié dans son rapport n/iso dans une étape de déshydrogénation (2) en un mélange de déshydrogénation (3) contenant du n-butène et de l'isobutène,
d) on conduit le mélange de déshydrogénation (3) à l'étape d'éthérification (4) et on transforme sélectivement l'isobutène avec un alcanol (5) en un terbutyléther d'alkyle (6), et
e) on conduit le mélange de déshydrogénation résiduel (7) à l'étape d'oligomérisation (8) et on oligomérise le n-butène par voie catalytique.

2. Procédé selon la revendication 1,
caractérisé en ce que
on dispose entre l'étape de déshydrogénation (2) et l'étape d'éthérification (4), ou entre l'étape d'éthérification (4) et l'étape d'oligomérisation (8), une étape d'hydrogénation sélective (14) et/ou une étape de purification (15) avec un tamis moléculaire.

3. Procédé selon l'une des revendications 1 ou 2,
caractérisé en ce qu'
une étape de séparation (16) sépare le n-butène du gaz résiduel I (17) après l'étape d'éthérification (4), à partir du mélange de déshydrogénation résiduel (7), et le gaz résiduel I est conduit à l'étape de déshydrogénation (2) ou à l'étape d'isomérisation (11), éventuellement par l'intermédiaire d'une étape d'hydrogénation (18).

4. Procédé selon l'une des revendications 1 à 3,
caractérisé en ce qu'
on sépare le gaz résiduel II (21) du mélange d'oligomérisation (19) dans une étape de séparation (20), et on le conduit à l'étape de déshydrogénation (2) ou à l'étape d'isomérisation (11), éventuellement par l'intermédiaire d'une étape d'hydrogénation (18).

5. Procédé selon l'une des revendications 1 à 4,
caractérisé en ce qu'
on utilise comme alcanol de l'éthanol, de l'isopropanol, de l'isobutanol ou en particulier du méthanol.
